# EUROPÄISCHE PATENTSCHRIFT

(11) **EP 0 401 199 B1**
(45) Veröffentlichungstag und Bekanntmachung des Hinweises auf die Patenterteilung: **25.01.1995**
(21) Anmeldenummer: 90890164.8
(22) Anmeldetag: 28.05.1990
(51) Int. Cl.: A61L 27/00

(54) **Verfahren zur Präparierung von biologischem Implantationsmaterial**
Method for the preparation of biological implant material
Méthode de préparation d'un matériau pour implant biologique

(30) Priorität: 31.05.1989 AT 1323/89
(43) Veröffentlichungstag der Anmeldung: 05.12.1990
(73) Patentinhaber: SORIN BIOMEDICA CARDIO S.p.A., I-10125 Torino (IT)
(72) Erfinder: Grimm, Michael, Dr., A-1180 Wien (AT); Eybl, Elisabeth, Dipl.-Ing., A-1030 Wien (AT); Griesmacher, Andrea, Dr., A-1170 Wien (AT); Grabenwöger, Martin, Dr., A-1130 Wien (AT); Müller, Mathias M., Dr., A-1040 Wien (AT); Wolner, Ernst, Dr., A-1140 Wien (AT)
(74) Vertreter: Bosotti, Luciano

(56) Entgegenhaltungen:
- FR-A- 2 306 707
- GB-A- 2 075 819

## Beschreibung

Die Erfindung bezieht sich auf ein Verfahren zur Präparierung von biologischem Implantationsmaterial, welches durch Aldehyde fixiert wurde, wobei der Überschuß an Aldehyden durch eine Aminodicarbonsäure gebunden wird.

Die chirurgische Implantation von biologischen Materialien ist eine häufig angewandte Technik und findet in vielen Gebieten der Medizin Anwendung. Die Herkunft dcs Materials kann dabei vom Empfänger selbst sein (autologes Gewebe), von der gleichen Spezies des Empfängers (homologes Gewebe), oder von einer anderen Spezies als der Empfänger (heterologes Gewebe). Beispiele für solche biologischen Materialien sind Herzklappen, Perikard, Sehnen, Bänder, Dura Mater, Knochen, Haut, Kollagen, Arterien, Venen und vieles mehr. Verschiedene Methoden, welche die Antigenität dieser Materialien reduzierten, machten es erst möglich, diese als Ersatz oder zur Reparatur bestimmter Defekte im Menschen zu implantieren, da es ohne entsprechende Vorbehandlung dieser biologischen Materialien zu Abstoßungsreaktionen im Menschen kommt. Diese Methoden haben hauptsächlich den Sinn, das Gewebe zu fixieren, d.h. zu denaturieren, zu welchem Zweck diese Materialien mit Aldehyden behandelt werden, z.B. mit Glutaraldehyd, Formaldehyd, Glyoxal oder anderen Aldehyden. Glutaraldehyd und Formaldehyd sind jedoch die am meisten Verwendeten Agenzien, da sie zusätzlich einen bekannt sterilisierenden Effekt haben. Außerdem sind sie für einen guten denaturierenden Effekt bekannt, d.h. sie reduzieren die Antigenität des Materials so weit, daß nach der Implantation keine Abstoßungsreaktionen mehr auftreten. Die in dem Gewebe vorhandenen Aldehydreste müssen jedoch soweit wie möglich entfernt werden, da die Aldehyde nach der Implantation starke Reizungen, z.B. entzündliche Reaktionen, oder andere schädliche Effekte hervorrufen können. Dazu werden üblicherweise die so behandelten Materialien vor der Implantation mit sterilen Lösungen gespült, z.B. Aqua bidest. oder mit isotonischen Kochsalzlösungen, um die Reste an freien, nicht gebundenen Aldehyden zu entfernen.

Bei einem Verfahren der eingangs genannten Art ist es auch schon bereits bekannt, die Aldehyde durch Zusatz von Aminodicarbonsäuren zu entfernen, wobei die Aldehydentfernungsreaktion in einem Milieu durchgeführt wird, dessen pH größer als 7 ist FR-A-2306707. Diese bekannten Verfahren beruhen dabei auf dem Prinzip, eine Schiffsche Basenreaktion zwischen freien, diffundierbaren Aldehyden im Gewebe und den NH2-Resten der zugesetzten Substanz zu erzielen. Da der Aldehyd mit steigendem pH auch eine steigende Tendenz zur Schiffschen Basenreaktion zeigt, wurde eben immer darauf geachtet, daß diese Reaktion in entsprechend neutralem bis alkalischen Milieu verläuft. Für diese Reaktionsmechanismen eignen sich außer den Aminocarbonsäuren auch noch primäre, sekundäre und tertiäre Amine sowie Gemische daraus.

Diese bekannten Ausbildungen haben jedoch den Nachteil, daß aufgrund des beschriebenen Reaktionsmechanismus eine nicht unbeachtliche Menge von Aldehyd im Gewebe zurückbleibt, wodurch nach wie vor Entzündungsreaktionen durch das implantierte Material hervorgerufen werden.

Der Erfindung liegt die Aufgabe zugrunde, ein Verfahren der eingangs genannten Art zu schaffen, mittels welchem die zur Fixierung benötigten Aldehyde soweit aus dem Material entfernt werden, daß sie keine Reizungsreaktionen des angrenzenden Gewebes mehr hervorrufen.

Diese Aufgabe wird erfindungsgemäß dadurch gelöst, daß die Behandlung des fixierten Implantationsmaterials mit der Aminodicarbonsäure in saurem Milieu, vorzugsweise in einem pH-Bereich von 2,5 bis 5,5, insbesondere 3 bis 5, durchgeführt wird. Dadurch wird die Bildung von Schiffschen Basen ausgeschlossen, da sich diese, wie schon vorstehend ausgeführt, nur in neutralem oder basischem pH-Bereich bilden können. Dies erklärt sich aus der Tatsache, daß positiv geladene Aminogruppen nicht zur Bildung Schiffscher Basen fähig sind (Houben-Weyl). Im Falle der vorliegenden Erfindung liegen die Aminogruppen der genannten Säuren aufgrund ihrer pKa-Werte in einem positiven Ladungszustand vor. Es bilden sich in diesem pH-Bereich dann anstelle der Schiffschen Basen N,O-Halbacetale, die sich bei einem Überschuß von Aminogruppen in N,N-Acetale umwandeln. Weiters werden die Halbacetale, die zwischen Kollagen (OH-Gruppen von Lysin, Threonin und Hydroxyprolin) und Glutaraldehyd ausgebildet haben, durch saure Katalyse in die stabileren Acetale übergeführt.

Wenn Glutaraldehyd zur Fixierung verwendet wird, dann ist auch noch zu bedenken, daß monomerer, linearer Glutaraldehyd in wässeriger Lösung im Gleichgewicht mit drei hydratisierten linearen bzw. zyklischen Verbindungen steht. Ferner sind auch verschiedene oligomere bzw. polymere Formen bekannt, die auf Aldolkondensationen bzw. auf wasserkatalysierte Polymerisation des Aldehyds zurückgeführt werden. Diese chemische Uneinheitlichkeit des Glutaraldehyds erklärt die komplexen Reaktionsmechanismen mit Proteinen, z.B. mit Kollagen. Die hohe Reaktivität des Glutaraldehyds erklärt sich durch einen Solvatationseffekt, durch eine günstige Kettenlänge zur Ausbildung intermolekularer Querbrücken und die mögliche Stabilisierung durch zyklische Monohydrate des Glutaraldehyds. Trotz einer Vielzahl von Untersuchungen konnte jedoch ein einheitlicher Reaktionsmechanismus von Glutaraldehyden mit Proteinen nicht geklärt werden. Die quervernetzende Wirkung von Glutaraldehyd auf Kollagen ist bekannterweise im alkalischen Milieu am höchsten, so daß die Quervernetzung von Proteinen durch Glutaraldehyd auf eine Ausbildung von Schiffschen Basen zurückgeführt wird. Die beschriebenen Polymerisationsprodukte sind zu einem Großteil an den toxischen Effekten aldehyd-modifizierter Biomaterialien beteiligt. Durch ihren erhöhten Polymerisationsgrad und durch die engmaschig vernetzte Kollagenstruktur ist ein effizientes Ausdiffundieren dieser Produkte nicht möglich, so daß sie hier nach der chirurgischen Implantation wieder wirksam werden können.

Der erfindungsgemäß eingesetzte niedrige pH-Wert spaltet die Polymerisate des Glutaraldehyds wieder in seine monomere Ausgangsform, was eine Reduktion der toxischen Polymerisationsprodukte mit sich führt und ein verstärktes Ausdiffundieren induziert. Weitere können durch die Aufspaltung der Polymerisationsprodukte alle freien, nicht vernetzenden Aldehydgruppen zur Reaktion mit den Aminodicarbonsäuren gebracht werden. Der Grund, warum sich für das erfindungsgemäße Reaktionsschema nur die genannten Aminodicarbonsäuren eignen, ist, daß diese Säuren zusätzlich mit dem Kollagen vernetzende und daher stabilisierende Bindungen eingehen, bei welchen es sich nicht um Schiffsche Basen handelt, sondern um Esterbildungen, die mit freien OH-Gruppen des Kollagens und der Säure entstehen, und um die schon vorher erwähnte Acetal- bzw. Halbacetalbildung. Weiters können die genannten Aminodicarbonsäuren mit den freien Resten des kollagenen Gewebes Salzbindungen eingehen, die in diesem Fall die Biokompatibilität der Oberfläche des implantierten Produktes stark erhöhen.

Die biologisch wirksame Toxizität der noch im Gewebe verbleibenden Reste an freiem, nicht gebundenen Glutaraldehyd kann in vitro mittels Zellgewebekulturen ausgetestet werden. Das biologische Gewebe wird nach bekannten Methoden entnommen, wie allgemein üblich präpariert und dann der Fixierung bzw. Sterilisierung zugeführt. Durch diese Tests kann eine erhebliche Verringerung von durch die Aldehyde hervorgerufenen toxischen Effekten festgestellt werden, wenn die Implantationsmaterialien nach dem erfindungsgemäßen Verfahren behandelt werden. Die Verringerung der toxischen Effekte ist allen bisher bekannten Methoden signifikant überlegen, wie dies später anhand von Testbeispielen nachgewiesen wird.

Da erfindungsgemäß Aminodicarbonsäuren eingesetzt werden, kann sich der gewünschte pH-Bereich dann von selbst einstellen, wenn die Aminodicarbonsäuren in sterilem Aqua dest. bzw. Aqua bidest. gelöst werden. Beim erfindungsgemäßen Verfahren können die Aminodicarbonsäuren (das sind Glutaminsäure bzw. Asparaginsäure bzw. deren Isomere und Gemische derselben) auch so eingesetzt werden, daß die Behandlung des fixierten Implantationsmaterials in einem gepufferten Medium vorgenommen wird, wobei als Puffersystem vorzugsweise eines aus der folgenden Gruppe eingesetzt wird: Natriumcitrat/HCl-, Citrat-Phosphat-Borat/HCl- oder Acetat-Puffer. Es können allerdings auch alle anderen Puffer verwendet werden, die keine Aminogruppe enthalten oder nicht toxische Reaktionen hervorrufen, welche dann die Wirksamkeit des erfindungsgemäßen Verfahrens beeinträchtigen würden.

Weiters kann bei einer Einwirkdauer der Aldehyde von etwa 72 Stunden die Behandlung mit der Aminodicarbonsäure wenigstens 3 Stunden bei konstanter Temperatur, insbesondere bei einer Temperatur zwischen 4° und 45° C vorgenommen werden. Dadurch wird eine effektive Verminderung der freien Aldehydgruppen im Gewebe erzielt, da genügend lange Einwirkzeit vorhanden ist, um ein Ausdiffundieren der im Gewebe vorhandenen Aldehydreste zu erzielen.

Die Menge an verwendet er Aminosäure kann dabei variieren, jedoch kann, besonders bevorzugt, in der Behandlungslösung eine Konzentration der Aminodicarbonsäuren von 0,001 M bis 2 M eingesetzt werden. Die Auswahl der verwendeten Konzentration hängt in großem Maß von der Durchführung der Postfixationsbehandlung ab, da eine enge Korrelation zwischen Konzentration der Aminosäurelösung, der Anzahl der Waschungen, der Einwirkdauer und dem Volumen der Aminodicarbonsäurelösung besteht. So würde z.B. eine Verkürzung der Einwirkzeit bei gleichbleibender Konzentration der Aminosäurelösung den Effekt der Toxizitätsverminderung der Aldehyde beeinträchten. Eine Konzentration an Aminosäuren von etwa einem zehnfachen Überschuß gegenüber den freien Aldehydgruppen ist in vorliegendem Fall wünschenswert.

Ein Vorteil des vorliegenden erfindungsgemäßen Verfahren liegt im übrigen auch noch darin, daß die verwendeten Aminodicarbonsäuren äußerst geringe Toxizität aufweisen, so daß das Implantationsmaterial direkt nach der Aminosäurebehandlung implantiert werden könnte. Es ist jedoch angezeigt, nach der Aminosäurebehandlung eine bzw. mehrere Waschungen in isotonen Lösungen vorzunehmen, um die Effektivität des vorliegenden erfindungsgemäßen Verfahrens aufrecht zu erhalten. Auch können noch irgendwelche Reste anderer Materialien, wie z.B. Puffer usw., aus dem Material herausgewaschen werden.

Da aufgrund des erfindungsgemäßen Verfahrens der gesamte Anteil an Aldehyden herausgewaschen wurde, kann das Implantationsmaterial nach der Behandlung mit Aminodicarbonsäure steril, vorzugsweise in einer biologisch verträglichen Sterilisationslösung wie Paraben gelagert werden. Dies ist dann der Fall, wenn nicht, wie schon angeführt, die Biomaterialien direkt steril implantiert werden. Das Manipulieren mit den biologischen Materialien soll daher ebenfalls unter sterilen Bedingungen stattfinden, am besten geeignet erweist sich eine Manipulation in einem Laminar Air Flow. Die Lagerung in einer sterilen Parabenlösung (0,02 % n-Propyl p-Hydroxy Benzoat und 0,18 % p-Hydroxybenzoesäure Methylester, gelöst in phosphatgepufferter Salzlösung) hat sich dabei deshalb als vorteilhaft erwiesen, weil diese Lösung keinerlei, die Bioverträglichkeit herabsetzende Wirkung aufweist. Für die Auswahl der Lagerlösung ist die Toxizität der hiefür verwendeten Chemikalien von entscheidender Bedeutung, da sie die Effektivität des vorliegenden erfindungsgemäßen Verfahrens erheblich beeinträchtigen können. Die Verwendung anderer Lagerlösungen als sterile 0,9 %ige Kochsalzlösungen setzt allerdings voraus, daß man die biologischen Materialien vor der chirurgischen Implantation wieder einer oder mehreren Waschungen unterzieht, um aus dem Gewebe eventuell biologisch unverträgliche Chemikalien der Lagerlösung zu entfernen.

Die vorliegende Erfindung wird nachstehend anhand von Verfahrensbeispielen näher erläutert, wobei in den aufscheinenden Tabellen die nach dem erfindungsgemäßen Verfahren behandelten Proben mit einem E gekennzeichnet sind.

Bei den Beispielen handelt es sich um Parallelversuche mehrerer bekannter Methoden, die parallel zu dem erfindungsgemäßen Verfahren mitgeführt wurden, um die Unterschiede zwischen den bekannten Verfahren und dem erfindungsgemäßen Verfahren deutlich hervorzukehren.

### Beispiel 1

Bovines Perikard von Kälbern wurde am lokalen Schlachthof gewonnen und sofort in eiskalter 0,9 % Kochsalzlösung gelagert. Binnen einer Stunde begann dan die Verarbeitung des Materials im Labor. Das Perikard wurde gründlich von überschüssigem Fett- bzw. Bindegewebe befreit, ein Vorgang, der ebenfalls unter eiskalter Kochsalzlösung vor sich ging. Das Perikard wurde dann in eine 0,5% Glutaraldehydlösung (pH 7,4) eingelegt und für 72 Stunden bei 4° C gelagert.

Nach diesem Zeitraum wurde das Material, unter sterilen Bedingungen im Laminar Air Flow, in 1 cm² große Stücke zerschnitten und in verschiedene Gruppen aufgeteilt.
1) Entweder wurde es sofort in die Lagerlösung (0,25 % GA-Lösung bzw. Parabenlösung) transferiert, um dort für mehrere Wochen gelagert zu werden, oder es wurde einer 24stündigen Postfixationsbehandlung zugeführt. Nach Abschluß dieser Postfixationsbehandlung wurden die solcherart behandelten Materialien in einer aldehydfreien Lösung gelagert. In diesem Fall war es eine Parabenlösung, die jedoch nicht Bestandteil dieser Erfindung ist.
   Verwendete Postfixationsbehandlungen:
   Die Behandlung erstreckte sich immer über einen Zeitraum von 24 Stunden, und alle Behandlungen wurden bei Raumtemperatur durchgeführt.
   a) phosphatgepufferte Saline (PBS) ohne Calcium und Magnesium
   b) 0,5 % Ammoniaklösung (NH3) in Aqua dest.
   c) foetales bovines Serum
   d) 4 % Guanidiniumchloridlösung (GCL) in PBS (pH 7,35)
   e) 4 % Guanidiniumchloridlösung (GCL) in PBS (pH 7,35), gefolgt von einer 10minütigen Behandlung in 1 % Natrium Borhydrid (NaBH4)
   f) 4 % Glycinlösung in PBS (pH 7,35)
   g) 4 % Glycinlösung in Aqua dest. (pH 3)
   h) 4 % Glutaminsäurelösung in PBS (pH 7,35)
   i) 4 % Glutaminsäurelösung in Aqua dest. (pH 3)
   j) 4 % Glutaminsäurelösung in PBS (pH 7,35), gefolgt von einer 10minütigen Behandlung in 1 % Natrium Borhydrid (NaBH4)
   k) 4 % Asparaginsäurelösung in PBS (pH 7,35)
   l) 4 % Asparaginsäurelösung in Aqua dest. (pH 3)
   i) und l) sind also erfindungsgemäße Verfahren.
2) Nach diesem Vorgang wurden die solcherart behandelten Biomaterialien für mindestens 1 Monat gelagert, um dann einem in vitro Test unterzogen zu werden.

In diesem Testverfahren wurde die Möglichkeit untersucht, bovine aortale Endothelzellen auf den Materialien zu kultivieren. Diese Methoden gibt genaueste Aufschlüsse über toxische Effekte, die von chemisch modifizierten Biomaterialien evoziert werden, da die Zellen in direktem Kontakt mit den Materialien kultiviert werden. Die Gewinnung und Kultivation von bovinen aortalen Endothelzellen ist eine Standardmethodik, die in der Literatur bereits seit längerer Zeit beschrieben ist. Vor der Zellaussaat wurden die Materialien verschiedenen Waschungen unterworfen, um die Lagerungslösungen zu entfernen.

Die verwendete Menge an Waschmedium pro 1 cm² großem Stück war 20 ml.
Als Waschmedien wurden verwendet:
0,9 % Kochsalzlösung (NaCl) Heilmittelwerke Wien
Humanes Serum
Foetales bovines Serum (Gibco)
Nach den diversen Waschungen wurden alle Klappensegel in 24-Lochplatten eingelegt und für 4 Stunden mit humanen Fibronektin vorbeschichtet. Danach wurden die Zellen mit einer primären Aussaatdichte von 35.300 +/-9.800 auf die Materialien aufgebracht. Nun wurden die zellen auf den Materialien für einen Zeitraum von 10 Tagen kultiviert. Am 3. und am 10 Tag nach der Aussaat wurde die Zellzahl bestimmt. Zu diesem Zweck wurden die zellen mittels Collagenase Typ I von den Materialien abgelöst, mit Kristallviolett angefärbt, und in einem Hämatozytocounter wurde die Zellzahl bestimmt, welche als Zellzahl/cm² angegeben ist. Parallel zu allen Versuchen wurden immer Kontrollen mitgeführt, wobei die Zellen unter Standardkulturbedingungen in der 24-Lochplatte kultiviert wurden.

Der Score TOX ist eine Beschreibung der toxischen Beeinflussung des Zellwachstums, zum Zeitpunkt der Zellzählung, und richtet sich nach Beobachtungen der Zellmembranintegrität im Rasterelektronenmikroskop.
- V: = sehr stark toxisch
- IV: = stark toxisch
- III: = mäßig toxisch
- II: = kaum toxisch
- I: = nicht toxisch
Jedes der genannten Materialien wurde in Doppelbestimmung (Tabelle 1 und Tabelle 2) in 5 verschiedenen Serien getestet, und die Zellzahl wird als Mittelwert angegeben.

**Tabelle 1**

| FIXIERUNG | NACHBEHANDLUNG | | LAGERUNG | WASCHUNG | Zellzahl (3.Tag) | TOX |
|---|---|---|---|---|---|---|
| 72h 0.5%GA | --- | | 0.25%GA | 3x10min NaCl | 0 | V |
| 72h 0.5%GA | --- | | 0.25%GA | 3x10min FBS | 0 | V |
| 72h 0.5%GA | --- | | 0.25%GA | 3x10min HS | 0 | V |
| 72h 0.5%GA | --- | | 0.25%GA | 3x1d NaCl | 0 | V |
| 72h 0.5%GA | --- | | 0.25%GA | 3x1d FBS | 1.035 | IV |
| 72h 0.5%GA | --- | | Paraben | 3x10min NaCl | 5.450 | IV |
| 72h 0.5%GA | PBS | pH7 | Paraben | 3x10min NaCl | 9.760 | IV |
| 72h 0.5%GA | FBS | | Paraben | 3x10min NaCl | 8.340 | IV |
| 72h 0.5%GA | 0.5%NH3 | | Paraben | 3x10min NaCl | 7.540 | IV |
| 72h 0.5%GA | 4%GCl | pH7 | Paraben | 3x10min NaCl | 25.670 | III |
| 72h 0.5%GA | 4%GCl | pH7 | Paraben | 3x10min NaCl | 24.900 | III |
| | 10min | 1%NaBH4 | | | | |
| 72h 0.5%GA | 4%Glycin | pH7 | Paraben | 3x10min NaCl | 34.070 | II |
| 72h 0.5%GA | 4%Glycin | pH3 | Paraben | 3x10min NaCl | 32.980 | II |
| 72h 0.5%GA | 4%GS | pH7 | Paraben | 3x10min NaCl | 29.870 | III |
| 72h 0.5%GA | 4%GS | pH3 | Paraben | 3x10min NaCl | 57.980 E | I |
| 72h 0.5%GA | 4%GS | pH7 | Paraben | 3x10min NaCl | 33.980 | II |
| | 10min | 1%NaBH4 | | | | |
| 72h 0.5%GA | 4%GS | pH9 | Paraben | 3x10min NaCl | 32.950 | II |
| 72h 0.5%GA | 4%AS | pH7 | Paraben | 3x10min NaCl | 34.070 | II |
| 72h 0.5%GA | 4%AS | pH3 | Paraben | 3x10min NaCl | 49.890 E | I |
| Zellzahl in paralell durchgeführten Kontrollen | | | | | 56.080 | I |

**Tabelle 2**

| FIXIERUNG | NACHBEHANDLUNG | | LAGERUNG | WASCHUNG | Zellzahl (10.Tag) | TOX |
|---|---|---|---|---|---|---|
| 72h 0.5%GA | --- | | 0.25%GA | 3x10min NaCl | 0 | V |
| 72h 0.5%GA | --- | | 0.25%GA | 3x10min FBS | 0 | V |
| 72h 0.5%GA | --- | | 0.25%GA | 3x10min HS | 0 | V |
| 72h 0.5%GA | --- | | 0.25%GA | 3x1d NaCl | 0 | V |
| 72h 0.5%GA | --- | | 0.25%GA | 3x1d FBS | 0 | V |
| 72h 0.5%GA | --- | | Paraben | 3x10min NaCl | 0 | V |
| 72h 0.5%GA | PBS | pH7 | Paraben | 3x10min NaCl | 0 | V |
| 72h 0.5%GA | FBS | | Paraben | 3x10min NaCl | 0 | V |
| 72h 0.5%GA | 0.5%NH3 | | Paraben | 3x10min NaCl | 0 | V |
| 72h 0.5%GA | 4%GCl | pH7 | Paraben | 3x10min NaCl | 0 | V |
| 72h 0.5%GA | 4%GCl | pH7 | Paraben | 3x10min NaCl | 0 | V |
| | 10min | 1%NaBH4 | | | | |
| 72h 0.5%GA | 4%Glycin | pH7 | Paraben | 3x10min NaCl | 0 | V |
| 72h 0.5%GA | 4%Glycin | pH3 | Paraben | 3x10min NaCl | 0 | V |
| 72h 0.5%GA | 4%GS | pH7 | Paraben | 3x10min NaCl | 0 | V |
| 72h 0.5%GA | 4%GS | pH3 | Paraben | 3x10min NaCl | 124.420 E | I |
| 72h 0.5%GA | 4%GS | pH7 | Paraben | 3x10min NaCl | 0 | V |
| | 10min | 1%NaBH4 | | | | |
| 72h 0.5%GA | 4%GS | pH9 | Paraben | 3x10min NaCl | 0 | V |
| 72h 0.5%GA | 4%AS | pH7 | Paraben | 3x10min NaCl | 0 | V |
| 72h 0.5%GA | 4%AS | pH3 | Paraben | 3x10min NaCl | 113.560 E | I |
| Zellzahl in paralell durchgeführten Kontrollen | | | | | 132.570 | I |

### Beispiel 2

Die 1 cm² großen Stücke von bovinem Perikard (für Fixierung, Nachbehandlung, Lagerung und Waschung siehe Beispiel 1) wurden, nach einer Kurznarkose, in 300 g schwere Sprauge Darley Ratten subcutan implantiert. Dabei wurden jeweils 4 verschiedene Materialien an der Bauchseite das Tieres implantiert, wobei die Zusammenstellung der Materialien wahllos war. Jedes Material wurde 7 mal implantiert. Nach einer Dauer von 63 Tagen wurden die Materialien explantiert, und mittels Atomabsorptionsspektroskopie wurde der Calciumgehalt der Proben bestimmt. Die Angabe erfolgt in g Calcium/mg Trockengewicht (TG) (s. Tabelle 3).

Aus jedem der Präparate wurde aus der Mitte ein kleines Stück Probe entnommen und einer histologischen Untersuchung zugeführt, wobei die Proben nach der Methode van Kossa, einer speziellen Calciumphosphatfärbung, gefärbt wurden. Im Lichtmikroskop wurden die Präparate dann untersucht, und ein Score HISTO für das quantitative und qualitative Auftreten von Calcium aufgestellt.
- III: = starke Verkalkung aller Schichten des Materials, mit Zerstörung von einem Großteil der kollagenen Matrix
- II: = Verkalkung der zentralen Anteile des Materials, mit Erhaltung von einem Großteil der kollagenen Matrix
- I: = kaum bis spärliches Auftreten von Verkalkung, ohne sichtbare Störung der Integrität der kollagenen Matrix

**Tabelle 3**

| FIXIERUNG | NACHBEHANDLUNG | | LAGERUNG | WASCHUNG | µgCALCIUM/mgTG | HISTO |
|---|---|---|---|---|---|---|
| 72h 0.5%GA | | | 0.25%GA | 3x10min NaCl | 169.05 ± 24.91 | III |
| 72h 0.5%GA | | | 0.25%GA | 3x1d NaCl | 173.03 ± 34.89 | III |
| 72h 0.5%GA | | | Paraben | 3x10min NaCl | 148.93 ± 36.98 | III |
| 72h 0.5%GA | 4%GCl | pH7 | Paraben | 3x10min NaCl | 178.45 ± 34.98 | III |
| 72h 0.5%GA | 4%Glycin | pH7 | Paraben | 3x10min NaCl | 157.34 ± 36.87 | III |
| 72h 0.5%GA | 4%Glycin | pH7 | Paraben | 3x10min NaCl | 180.03 ± 67.89 | III |
| | 10min | 1%NaBH4 | | | | |
| 72h 0.5%GA | 4%Glycin | pH3 | Paraben | 3x10min NaCl | 189.38 ± 45.57 | III |
| 72h 0.5%GA | 4%GS | pH7 | Paraben | 3x10min NaCl | 145.78 ± 36.78 | III |
| 72h 0.5%GA | 4%GS | pH3 | Paraben | 3x10min NaCl | 13.65 ± 10.41 | I E |
| 72h 0.5%GA | 4%GS | pH7 | Paraben | 3x10min NaCl | 226.78 ± 78.48 | III |
| | 10min | 1%NaBH4 | | | | |
| 72h 0.5%GA | 4%GS | pH9 | Paraben | 3x10min NaCl | 134.96 ± 36.78 | III |
| 72h 0.5%GA | 4%AS | pH7 | Paraben | 3x10min NaCl | 156.89 ± 17.07 | III |
| 72h 0.5%GA | 4%AS | pH3 | Paraben | 3x10min NaCl | 29.23 ± 7.05 | I E |

## Patentansprüche

1. Verfahren zur Präparierung von biologischem Implantationsmaterial, welches durch Aldehyde fixiert wurde, wobei der überschuß an Aldehyden durch eine Aminodicarbonsäure gebunden wird, dadurch gekennzeichnet, daß die Behandlung des fixierten Implantationsmaterials mit der Aminodicarbonsäure in saurem Milieu, vorzugsweise in einem pH-Bereich von 2,5 bis 5,5, insbesondere 3 bis 5, durchgeführt wird.

2. Verfahren nach Anspruch 1, dadurch gekennzeichnet, daß die Behandlung des fixierten Implantationsmaterials in einem gepufferten Medium vorgenommen wird, wobei als Puffersystem vorzugsweise eines aus der folgenden Gruppe eingesetzt wird: Natriumcitrat/HCl-, Citrat-Phosphat-Borat/HCl- oder Acetatpuffer.

3. Verfahren nach Anspruch 1, dadurch gekennzeichnet, daß zur Einstellung des sauren Bereiches der Behandlung des fixierten Implantationsmaterials die eingesetzte Aminodicarbonsäure in wässeriger Lösung eingesetzt wird.

4. Verfahren nach einem der Ansprüche 1 bis 3, dadurch gekennzeichnet, daß bei einer Einwirkdauer der Aldehyde von etwa 72 Stunden die Behandlung mit der Aminodicarbonsäure wenigstens drei Stunden bei konstanter Temperatur, insbesondere bei einer Temperatur zwischen 4 und 45° C, vorgenommen wird.

5. Verfahren nach einem der Ansprüche 1 bis 4, dadurch gekennzeichnet, daß in der Behandlungslösung eine Konzentration der Aminodicarbonsäure von 0,001 M bis 2 M eingestellt wird.

6. Verfahren nach einem der Ansprüche 1 bis 5, dadurch gekennzeichnet, daß das Implantationsmaterial nach der Behandlung mit der Aminodicarbonsäure steril, vorzugsweise in einer biologisch verträglichen Sterilisationslösung wie Paraben, gelagert wird.

## Claims

1. A method of preparing biological implantation material fixed with an aldehyde wherein the aldehyde excess is bonded with an aminodicarboxylic acid, characterised in that treatment of the fixed implantation material takes place in an acidic medium, preferably in a pH - range from 2.5 to 5.5, particularly from 3 to 5.

2. The method of claim 1 characterised in that treatment of the fixed implantation material is conducted in a buffered medium, wherein one of the following groups is used as the buffer system: sodium citrate/HCl-citratephosphateborate/HCl and acetate buffer.

3. The method according to claim 1, characterised in that in order to adjust the acidic range of treatment of the fixed implantation material, the aminodicarboxylic acid used is used in an aqueous solution.

4. The method according to any of claims 1 to 3, characterised in that if the aldehydes are allowed to react for about 72 hours, the treatment with the aminocarboxylic acid is carried out for at least three hours at a constant temperature, particularly at a temperature between 4°C and 45°C.

5. The method according to any of claims 1 to 4, characterised in that a concentration of the aminocarboyxlic acid from 0.001 M to 2.0 M is adjusted in the treatment solution.

6. The method according to any of claims 1 to 5, characterised in that after treatment with aminocarboxylic acid the implantation material is stored under sterile conditions, preferably in a biologically tolerated sterilization solution such as Paraben.

## Revendications

1. Procédé de préparation d'un matériau pour implant biologique, ayant été fixé par un aldéhyde, l'aldéhyde excédentaire étant lié à un acide aminodicarboxylique caractérisé en ce que le traitement du matériau d'implant fixé en combinaison avec l' acide aminodicarboxylique s'effectue en milieu acide, présentant de préférence un pH situé entre 2,5 et 5,5, en particulier entre 3 et 5.

2. Procédé selon la revendication 1, caractérisé en ce que le traitement du matériau d'implant fixé est réalisé dans un milieu tamponné, un élément du groupe suivant : citrate de sodium/HCl, citrate-phosphateborate/HCl ou acétate étant, de préférence, employé comme système tampon.

3. Procédé selon la revendication 1, caractérisé en ce que, pour ajuster la plage d'acidité pour le traitement du matériau d'implant fixé, l' acide aminodicarboxylique utilisé est employé sous forme de solution aqueuse.

4. Procédé selon l'une des revendications 1 à 3, caractérisé en ce que, lorsque l'aldéhyde a une durée d'action d'environ 72 heures, le traitement par l' acide aminodicarboxylique dure au moins trois heures à température constante, en particulier à une température située entre 4 et 45°C.

5. Procédé selon l'une des revendications 1 à 4, caractérisé en ce qu'une concentration d' acide aminodicarboxylique située entre 0,001 M et 2 M est prévue dans la solution de traitement.

6. Procédé selon l'une des revendications 1 à 5, caractérisé en ce que le matériau d'implant, à l'issue du traitement à l'acide aminodicarboxylique, est conservé de façon stérile, de préférence dans une solution de stérilisation biologiquement compatible, telle que des parahydroxybenzoates.
